# EUROPÄISCHE PATENTSCHRIFT

(11) **EP 3 233 810 B1**
(45) Veröffentlichungstag und Bekanntmachung des Hinweises auf die Patenterteilung: **11.09.2019**
(21) Anmeldenummer: 15817115.7
(22) Anmeldetag: 10.12.2015
(51) Int. Cl.: C07D 239/52, A01N 47/36, A01P 13/00, A01P 21/00

(54) **NEUE KRISTALLFORMEN DES MONONATRIUM-SALZES VON FORAMSULFURON**
NOVEL CRYSTAL FORMS OF THE MONOSODIUM SALT OF FORAMSULFURON
NOUVELLES FORMES DE CRISTAL DU SEL MONOSODIQUE DE FORAMSULFURON

(30) Priorität: 15.12.2014 EP 14198010
(43) Veröffentlichungstag der Anmeldung: 25.10.2017
(73) Patentinhaber: Bayer CropScience Aktiengesellschaft, 40789 Monheim am Rhein (DE)
(72) Erfinder: ANTONS, Stefan, 51373 Leverkusen (DE); OLENIK, Britta, 46242 Bottrop (DE); KRÜGER, Martin, 35325 Mücke (DE)
(74) Vertreter: BIP Patents
(86) Internationale Anmeldenummer: PCT/EP2015/079283
(87) Internationale Veröffentlichungsnummer: WO 2016/096613

(56) Entgegenhaltungen:
- EP-A1- 1 902 618
- WO-A1-95/29899

## Beschreibung

Die vorliegende Erfindung betrifft eine neue Kristallform des Mononatrium-Salzes von Foramsulfuron, ein Verfahren zur Herstellung dieser Kristallform, ihre Verwendung in agrochemischen Zubereitungen sowie bestimmte Zusammensetzungen, Mischungen bzw. agrochemischen Zubereitungen enthaltend diese Kristallform, sowie ein neues Mono-Methanol-Solvat des Mononatrium-Salzes von Foramsulfuron.

Foramsulfuron der nachfolgenden Formel (I) und dessen Mononatrium-Salz sind aus EP 0 757 679 A1 bekannt.

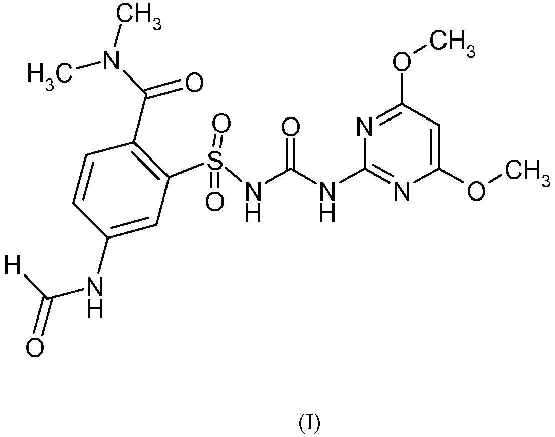

Foramsulfuron der Formel (I) und bestimmte Salze dieser Verbindung sind bekannte herbizide Wirkstoffe, siehe "The Pesticide Manual", 16th edition, The British Crop Protection Council and the Royal Society of Chemistry, 2012.

Ferner ist bekannt, dass Foramsulfuron der Formel (I) und dessen Salze in Kombination mit anderen herbiziden Wirkstoffen und/oder mit Safenern eingesetzt werden kann, wie beispielsweise beschrieben in EP 0 790 771 A1 oder EP 1 104 239 A1.

Foramsulfuron hat die Summenformel C₁₇H₂₀N₆O₇S, die CAS-Nummer 173159-57-4 und den IUPAC-Namen 1-(4,6-dimethoxypyrimidin-2-yl)-3-[2-(dimethylcarbamoyl)-5-formamidophenylsulfonyl]urea, und wird nachfolgend auch als N-[(4,6-Dimethoxypyridin-2-yl)-aminocarbonyl]-2-dimethylaminocarbonyl-5-formylamino-benzolsulfonamid bezeichnet.

Die Verbindung der Formel (I) und dessen Mononatrium-Salz können durch die in EP 0 757 679 A1 und US 6,500,952 B1 beschriebenen Verfahren hergestellt werden.

EP 1 902 618 A1 beschreibt Verfahren zur Herstellung von Sulfonamidsalzen.

Bislang war lediglich eine Kristallform des Mononatrium-Salzes von Foramsulfuron bekannt, die nachfolgend als Kristallform A bezeichnet wird. Diese Kristallform A entspricht einem Semi-Methanol-Solvat.

Das Kristallgitter eines Feststoffes kann auch aus mehreren chemischen Komponenten aufgebaut sein. Wenn eine davon eine Flüssigkeit (z.B. ein Lösungsmittel) ist, spricht man von Solvaten.

Das Auftreten von Wirkstoffen in verschiedenen kristallinen Modifikationen (Polymorphie) ist sowohl für die Ausarbeitung von Herstellungsverfahren als auch für die Entwicklung von Formulierungen (agrochemischen Zubereitungen) von großer Bedeutung. So unterscheiden sich die verschiedenen kristallinen Modifikationen einer chemischen Verbindung neben dem Aussehen (Kristallhabitus) und der Härte auch in zahlreichen weiteren physiko-chemischen Eigenschaften. Dabei können Unterschiede bezüglich der Stabilität, der Filtrierbarkeit, der Löslichkeit, der Hygroskopizität, des Schmelzpunktes, der Feststoffdichte und der Fließfähigkeit einen starken Einfluss auf die Qualität und die Wirksamkeit von Pflanzenbehandlungsmitteln ausüben. Es ist bisher nicht möglich, das Auftreten und die Anzahl von kristallinen Modifikationen einschließlich ihrer physiko-chemischen Eigenschaften vorherzusagen. Vor allem die thermodynamische Stabilität und auch das unterschiedliche Verhalten nach Darreichung in lebenden Organismen lassen sich nicht vorhersagen. Die vorstehenden Ausführungen zu Polymorphie gelten analog für Pseudopolymorphie.

Die bekannte Kristallform A weist einige nachteilige Eigenschaften auf, die nachfolgend kurz beschrieben werden.

Die Kristallform A kann sich nachteilig auf die Stabilität bzw. Lagerungsfähigkeit von Zusammensetzungen (z.B. von agrochemische Formulierungen) enthaltend die Kristallform A auswirken. Dies kann der Fall sein, weil z.B. die chemische Stabilität der Kristallform A in bestimmten Zusammensetzungen nicht ausreichend gegeben ist und/oder weitere Bestandteile (wie z.B. andere Wirkstoffe) dieser Zusammensetzungen durch die Kristallform A in ihrer chemischen Stabilität negativ beeinflusst werden und ihr Gehalt in der Zusammensetzung mit der Zeit abnimmt. Es kann auch vorkommen, dass eine Zusammensetzung (z.B. agrochemische Formulierung) enthaltend die Kristallform A über einen längeren (Lagerungs)Zeitraum nicht stabil ist, und sich die physikalischen Eigenschaften der Zusammensetzung verändert, sich beispielsweise die Viskosität bzw. Fließfähigkeit der Zusammensetzung in unerwünschter Weise ändert.

Ferner ist bekannt, dass Sulfonylharnstoff-Herbizide in Gegenwart bestimmter Hilfsstoffe, wie beispielsweise alkoholischen Verdünnungsmitteln, dabei insbesondere Methanol, chemische Abbaureaktionen unterliegen, was bei längeren Lagerungszeiträumen dazu führt, dass der Wirkstoffgehalt der Sulfonylharnstoff-Herbizide abnimmt, was unerwünscht ist. Dieser chemische Abbau tritt auch auf in Zusammensetzungen (wie beispielsweise Mischungen oder Formulierungen) enthaltend Foramsulfuron und dessen Salze in Kombination mit anderen Sulfonylharnstoff-Herbiziden bzw. deren Salze, beispielsweise bei den in EP 1 104 239 A1 beschriebenen Zusammensetzungen. Dies gilt insbesondere für chemisch leichter abbaubare / hydrolysierbare Sulfonylharnstoff-Herbizide wie Iodosulfuron, Iodosulfuron-methyl, und deren jeweiligen Salze, wie beispielsweise Iodosulfuron-Natrium oder Iodosulfuron-methyl-Natrium.

Ferner ist in manchen Fällen ist die technische Handhabung der bekannten Kristallform A schwierig. So fällt diese Kristallform A gemäß dem in EP 0 757 679 A1 beschriebenen Herstellungsverfahren in Form flockiger und sehr schlecht filtrierbarer Aggregate an.

Der Erfindung liegt daher die Aufgabe zugrunde, einem, mehreren oder sämtlichen der vorstehend im Zusammenhang mit der bekannten Kristallform A genannten Nachteil(en) abzuhelfen bzw. die vorstehend im Zusammenhang mit der bekannten Kristallform A genannten nachteiligen Eigenschaften zu verbessern.

Diese Aufgabe wird erfindungsgemäß durch eine neue Kristallform des Mononatrium-Salzes der Verbindung der Formel (I) mit der Summenformel C₁₇H₁₉N₆O₇SNa gelöst, die nachfolgend als Kristallform B bezeichnet wird.

Primär betrifft die Erfindung daher das Mononatrium-Salz von Foramsulfuron in kristalliner Form (Kristallform B), dadurch gekennzeichnet, dass das Röntgen-Pulverdiffraktogramm dieses Salzes bei Verwendung von Cu Kα-Strahlung bei 25°C mindestens 3 der folgenden 2Θ (2 Theta)-Werte aufweist:

| 2Θ (2Theta)-Werte in° |
|---|
| 6.2 |
| 6.4 |
| 14.4 |
| 14.6 |
| 14.7 |
| 18.4 |
| 19.2 |
| 20.1 |
| 23.2 |
| 24.7 |

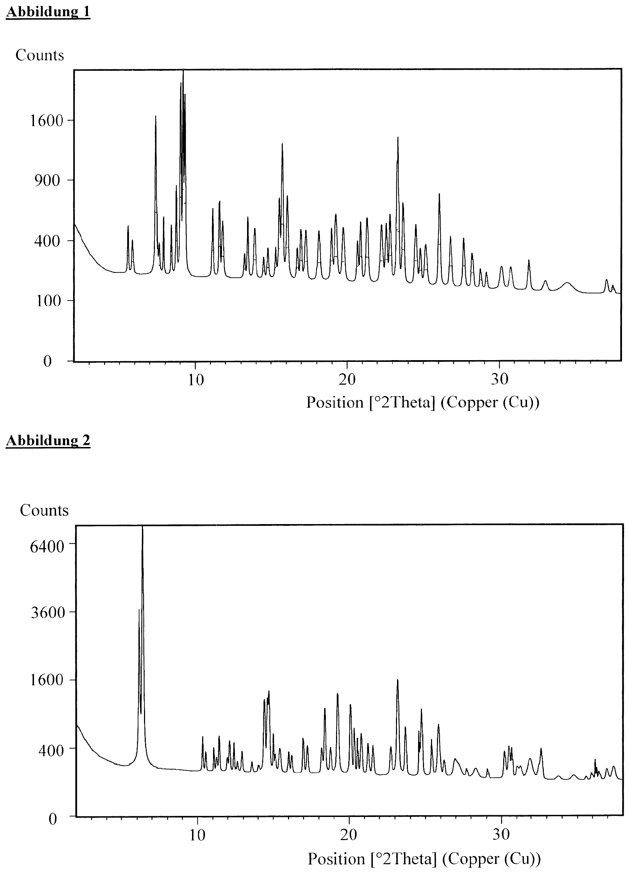

Diese erfindungsgemäße Kristallform B ist dadurch gekennzeichnet, dass sie ein Röntgenpulverdiffraktogramm mit den in Tabelle 1 angegebenen Reflexlagen 2Θ (2 Theta) aufweist. Das Röntgenpulverdiffraktogramm der Kristallform B ist auch in der Abbildung 2 wiedergegeben. Die intensivsten und charakteristischen Signale 2Θ (2 Theta) des Röntgenpulverdiffraktogramms der Kristallform B liegen bei 6.2, 6.4, 14.4, 14.6, 14.7, 18.4, 19.2, 20.1, 23.2, und 24.7° (jeweils ± 0,2°).

Vorzugsweise ist die erfindungsgemäße Kristallform B dadurch gekennzeichnet, dass das Röntgen-Pulverdiffraktogramm dieses Salzes bei Verwendung von Cu Kα-Strahlung bei 25 °C mindestens 4, vorzugsweise mindestens 5, bevorzugt mindestens 6, weiter bevorzugt mindestens 7, besonders bevorzugt mindestens 8, und insbesondere bevorzugt sämtliche, der vorstehend angegebenen intensivsten Signale 2Θ (2 Theta) aufweist.

Bevorzugt ist die erfindungsgemäße Kristallform B dadurch gekennzeichnet, dass das Röntgen-Pulverdiffraktogramm dieses Salzes bei Verwendung von Cu Kα-Strahlung bei 25 °C zusätzlich mindestens zwei, vier, sechs, acht, zehn, zwölf oder mehr, vorzugsweise mindestens 15, 20, 23, 25, 27, 30, 33, 35, 38, 40, 42, 44, 46, 48, 50 oder mehr, bevorzugt sämtliche, der folgenden 2Θ (2 Theta)-Werte aufweist:

| |
|---|
| 10.3 |
| 10.5 |
| 11.1 |
| 11.3 |
| 11.4 |
| 12.0 |
| 12.1 |
| 12.4 |
| 12.6 |
| 12.9 |
| 13.6 |
| 14.0 |
| 15.0 |
| 15.1 |
| 15.4 |
| 16.0 |
| 16.2 |
| 17.0 |
| 17.2 |
| 18.2 |
| 18.8 |
| 20.3 |
| 20.6 |
| 20.8 |
| 21.2 |
| 21.6 |
| 22.7 |
| 23.7 |
| 24.6 |
| 25.4 |
| 25.9 |
| 26.2 |
| 26.9 |
| 27.1 |
| 27.7 |
| 28.3 |
| 29.0 |
| 30.2 |
| 30.5 |
| 30.7 |
| 31.0 |
| 31.2 |
| 31.9 |
| 32.5 |
| 32.6 |
| 33.7 |
| 34.8 |
| 35.6 |
| 35.9 |
| 36.2 |
| 36.4 |
| 36.9 |
| 37.4 |

Die erfindungsgemäße Kristallform B ist, anders als die nachstehend beschriebene Kristallform C, nicht hygroskopisch und weist verbesserte Stabilitäten in Formulierungen (agrochemischen Zubereitungen) auf.

Die erfindungsgemäße Kristallform B ist besser geeignet zur Herstellung von (lager)stabilen Formulierungen, und sie ist besser kompatibel mit anderen Sulfonylharnstoff-Herbiziden und deren Salzen, insbesondere mit Iodosulfuron und dessen Salzen, da Sulfonylharnstoff-Herbizide (dabei insbesondere Iodosulfuron) und deren Salze eine nicht zufriedenstellende Stabilität, insbesondere eine nicht zufriedenstellende chemische Stabilität, in Mischung mit der Kristallform A aufweisen.

Alle im vorliegenden Text angegebenen Röntgenpulverdiffraktometrie-Daten beziehen sich auf die folgenden Messparameter:

| | |
|---|---|
| Scan Achse | Gonio |
| Scan Modus | Transmission |
| Start-Position [°2Theta] | 2.0066 |
| End-Position [°2Theta] | 37.9906 |
| Anodenmaterial | Cu |
| Wellenlänge K-Alphal [Å] | 1.54060 |
| Wellenlänge K-Alpha2 [Å] | 1.54443 |
| Wellenlänge K-Beta [Å] | 1.39225 |
| K-A2 / K-A1 Verhältnis | 0.50000 |
| Generator | 40 mA, 40 kV |
| Incident Beam Monochromator | focusing x-xay mirror |
| Spinning | Ja |
| Angabe 2Θ (2 Theta)-Werte | ± 0,2° |

Ferner wurde im Rahmen der vorliegenden Erfindung die nachfolgend als Kristallform C bezeichnete kristalline Form gefunden. Es handelt sich dabei im das Mono-Methanol-Solvat des Mononatrium-Salzes der Verbindung der Formel (I). Die Kristallform C kann beispielsweise erhalten werden, wenn die Kristallform A über einen längeren Zeitraum mit Methanol gelagert wird; dabei wandelt sich die Kristallform A in die Kristallform C um. Die Kristallform C wird im Detail weiter unten beschrieben.

Die Kristallformen A, B und C zeichnen sich durch unterschiedliche Röntgenpulverdiffraktogramme aus. In der nach folgenden Tabelle 1 sind die jeweils intensivsten, charakteristischen Reflexlagen 2Θ (2 Theta) angegebenen, die weiter unten stehende Tabelle 3 zeigt alle Reflexlagen 2Θ (2 Theta).

**Tabelle 1: Charakteristische Reflexlagen - Röntgendiffraktometrie der Kristallformen A, B und C (wie oben definiert)**

| Peakmaximum [2 Theta], Angabe in ° | | |
|---|---|---|
| Kristallform A | Kristallform B | Kristallform C |
| 7.4 | 6.2 | 9.1 |
| 8.7 | 6.4 | 9.1 |
| 9.0 | 14.4 | 9.7 |
| 9.2 | 14.6 | 12.6 |
| 9.3 | 14.7 | 16.2 |
| 15.7 | 18.4 | 18.5 |
| 23.3 | 19.2 | 24.1 |
| 23.3 | 20.1 | 24.3 |
| 23.6 | 23.2 | 24.6 |
| 26.0 | 24.7 | |

Sofern die im vorliegenden Text angegebenen Reflexlagen 2Θ (2 Theta) der Röntgenpulverdiffraktogramme für eine gegebene kristalline Form zwei mal denselben Wert aufweisen (wie beispielsweise das 2Θ-Peakmaximum bei 23.3° im Falle der Kristallform A), so handelt es sich dabei um zwei eng beieinander liegende Peaks. Durch Rundung des Messwertes des jeweiligen Peakmaximums auf eine Nachkommastelle können sich zwei Peaks mit demselben Wert ergeben, in dem Röntgenpulverdiffraktogramm handelt es sich jedoch tatsächlich um zwei Peaks.

Das Röntgenpulverdiffraktogramm der Kristallform A ist in Abbildung 1 wiedergegeben.

Das Röntgenpulverdiffraktogramm der Kristallform B ist in Abbildung 2 wiedergegeben.

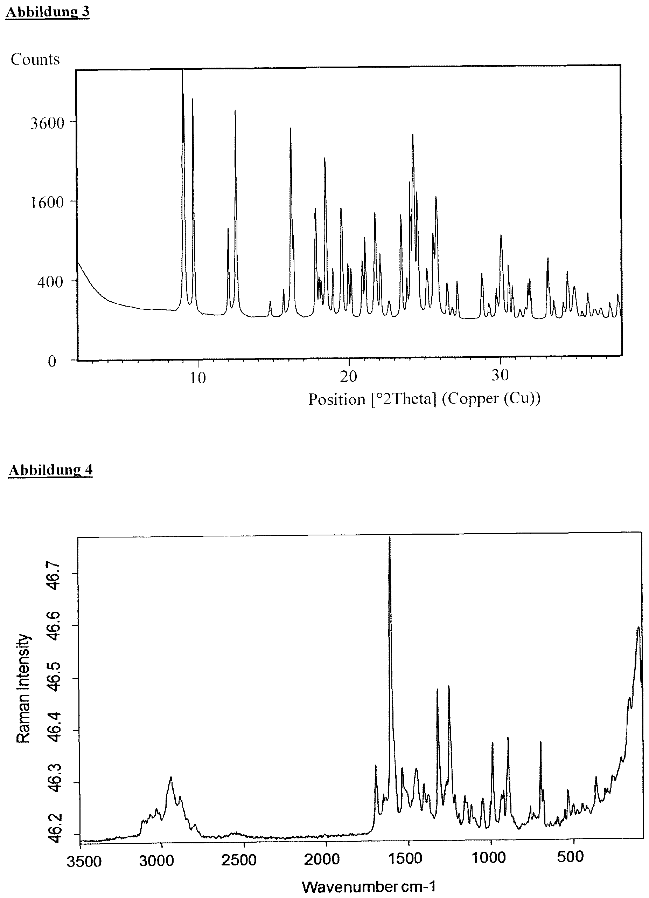

Das Röntgenpulverdiffraktogramm der Kristallform C ist in Abbildung 3 wiedergegeben.

Besonders bevorzugt ist das erfindungsgemäße Mononatrium-Salz von Foramsulfuron in kristalliner Form dadurch gekennzeichnet, dass das Röntgen-Pulverdiffraktogramm bei Verwendung von Cu Kα-Strahlung bei 25 °C im Wesentlichen dem in Abbildung 2 wiedergegebenen Spektrum entspricht.

Die Kristallformen A, B und C zeichnen sich durch unterschiedliche Raman-Spektren aus. In der nach folgenden Tabelle 2 sind die jeweils intensivsten, charakteristischen Banden angegebenen.

Alle im vorliegenden Text angegebenen Ramanspektroskopie-Daten beziehen sich auf die folgenden Messparameter, wobei die Lage der Bandenmaxima der Wellenzahl (englisch: Wavenumber) jeweils in cm⁻¹ angegeben sind:

| | |
|---|---|
| Gerät | Bruker Raman RFS 100/S |
| Anzahl Scans | 64 |
| Auflösung | 2 - 4 cm⁻¹ |
| Laser Power | 50 mW |
| Laser Wellenlänge | 1064 nm |

Bevorzugt ist die erfindungsgemäße Kristallform B dadurch gekennzeichnet, dass ihr Raman-Spektrum mindestens folgende Banden aufweist:

| Bandenmaximum [cm⁻¹] |
|---|
| 2959 |
| 2915 |
| 1686 |
| 1587 |
| 1526 |
| 694 |
| 345 |
| 133 |

Bevorzugt weist das Raman-Spektrum der erfindungsgemäßen Kristallform B mindestens zehn der in untenstehender Tabelle 4 angegebenen Banden auf, vorzugsweise 15, 20, 25, 30, 35, 40, 45, 50, 55, 60, 65 oder mehr, bevorzugt sämtliche, der in der untenstehenden Tabelle 4 angegebenen Banden.

**Tabelle 2: Charakteristische Banden der Ramanspektren der Kristallformen A, B und C (wie oben definiert)**

| Bandenmaximum [cm⁻¹] | | |
|---|---|---|
| Kristallform A | Kristallform B | Kristallform C |
| 2941 | 2959 | 3090 |
| 1700 | 2915 | 2943 |
| 1320 | 1686 | 2919 |
| 1250 | 1587 | 1593 |
| 990 | 1526 | 1576 |
| 697 | 694 | 691 |
| 362 | 345 | 156 |
| 103 | 133 | 122 |

Das Raman-Spektrum der Kristallform A ist in Abbildung 4 wiedergegeben.

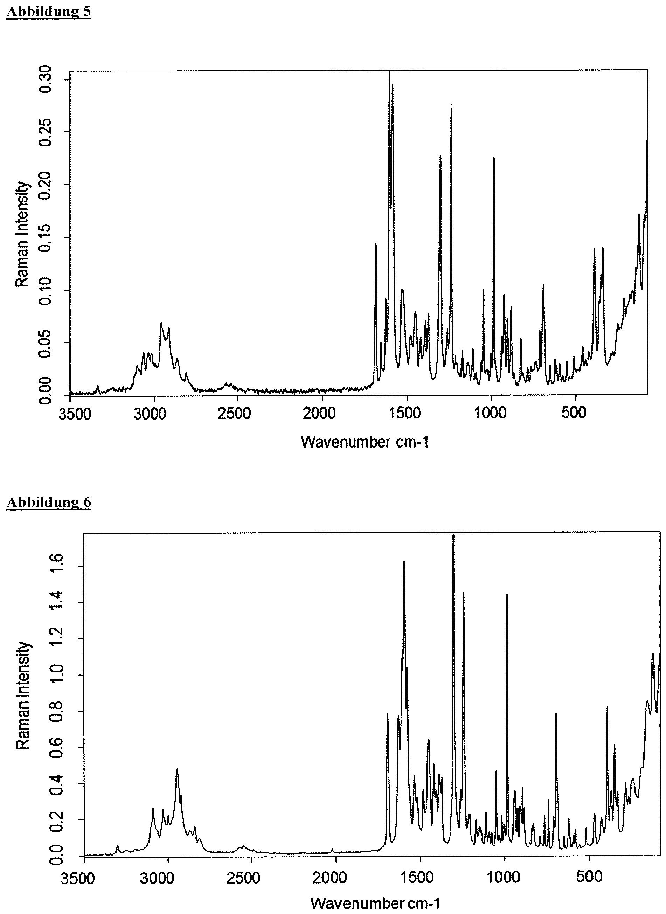

Das Raman-Spektrum der Kristallform B ist in Abbildung 5 wiedergegeben.

Das Raman-Spektrum der Kristallform C ist in Abbildung 6 wiedergegeben. Bevorzugt entspricht das Raman-Spektrum der erfindungsgemäßen Kristallform B im Wesentlichen dem in Abbildung 5 wiedergegebenen Spektrum.

**Tabelle 3: Röntgendiffraktometrie der Kristallformenen A, B und C (wie oben definiert)**

| Peakmaximum [2 Theta] | | |
|---|---|---|
| Kristallform A | Kristallform B | Kristallform C |
| 5.6 | 6.2 | 9.1 |
| 5.9 | 6.4 | 9.1 |
| 7.4 | 10.3 | 9.7 |
| 7.6 | 10.5 | 12.0 |
| 7.9 | 11.1 | 12.6 |
| 8.4 | 11.3 | 14.8 |
| 8.7 | 11.4 | 15.7 |
| 9.0 | 12.0 | 16.2 |
| 9.2 | 12.1 | 16.3 |
| 9.3 | 12.4 | 17.8 |
| 11.1 | 12.6 | 18.0 |
| 11.6 | 12.9 | 18.2 |
| 11.8 | 13.6 | 18.5 |
| 13.2 | 14.0 | 19.0 |
| 13.4 | 14.4 | 19.5 |
| 13.9 | 14.6 | 19.9 |
| 14.5 | 14.7 | 20.1 |
| 14.8 | 15.0 | 20.9 |
| 15.3 | 15.1 | 21.1 |
| 15.5 | 15.4 | 21.7 |
| 15.7 | 16.0 | 22.1 |
| 16.1 | 16.2 | 22.6 |
| 16.7 | 17.0 | 23.5 |
| 17.0 | 17.2 | 23.8 |
| 17.3 | 18.2 | 24.1 |
| 18.1 | 18.4 | 24.3 |
| 19.0 | 18.8 | 24.5 |
| 19.2 | 19.2 | 24.6 |
| 19.7 | 20.1 | 25.1 |
| 20.7 | 20.3 | 25.6 |
| 20.9 | 20.6 | 25.8 |
| 21.3 | 20.8 | 26.5 |
| 22.3 | 21.2 | 26.8 |
| 22.6 | 21.6 | 27.1 |
| 22.8 | 22.7 | 28.8 |
| 23.3 | 23.2 | 29.2 |
| 23.3 | 23.7 | 29.7 |
| 23.6 | 24.6 | 30.0 |
| 24.5 | 24.7 | 30.5 |
| 24.8 | 25.4 | 30.8 |
| 25.1 | 25.9 | 31.3 |
| 26.0 | 26.2 | 31.7 |
| 26.8 | 26.9 | 31.9 |
| 27.6 | 27.1 | 32.0 |
| 28.2 | 27.7 | 33.1 |
| 28.7 | 28.3 | 33.2 |
| 29.1 | 29.0 | 33.5 |
| 30.1 | 30.2 | 34.2 |
| 30.7 | 30.5 | 34.4 |
| 32.0 | 30.7 | 34.9 |
| 33.0 | 31.0 | 35.4 |
| 34.4 | 31.2 | 35.8 |
| 37.0 | 31.9 | 36.2 |
| 37.4 | 32.5 | 36.6 |
| | 32.6 | 37.2 |
| | 33.7 | 37.7 |
| | 34.8 | |
| | 35.6 | |
| | 35.9 | |
| | 36.2 | |
| | 36.4 | |
| | 36.9 | |
| | 37.4 | |

**Tabelle 4: Ramanspektroskopie-Banden der Kristallformen A, B und C (wie oben definiert)**

| Bandenmaximum [cm⁻¹] | | |
|---|---|---|
| Kristallform A | Kristallform B | Kristallform C |
| 3114 | 3338 | 3302 |
| 3097 | 3100 | 3090 |
| 3072 | 3061 | 3030 |
| 3032 | 3035 | 3016 |
| 2941 | 3018 | 2999 |
| 2890 | 2959 | 2943 |
| 2878 | 2915 | 2919 |
| 2842 | 2865 | 2870 |
| 2798 | 2810 | 2839 |
| 1700 | 1686 | 2812 |
| 1691 | 1654 | 1693 |
| 1653 | 1628 | 1628 |
| 1639 | 1604 | 1617 |
| 1604 | 1587 | 1607 |
| 1537 | 1526 | 1593 |
| 1513 | 1478 | 1576 |
| 1449 | 1451 | 1533 |
| 1404 | 1421 | 1517 |
| 1382 | 1393 | 1480 |
| 1320 | 1372 | 1449 |
| 1267 | 1304 | 1415 |
| 1250 | 1262 | 1404 |
| 1218 | 1244 | 1384 |
| 1196 | 1216 | 1372 |
| 1160 | 1206 | 1303 |
| 1150 | 1175 | 1261 |
| 1120 | 1142 | 1243 |
| 1101 | 1112 | 1215 |
| 1053 | 1092 | 1207 |
| 1004 | 1064 | 1168 |
| 990 | 1048 | 1148 |
| 936 | 1031 | 1137 |
| 926 | 1022 | 1111 |
| 896 | 1002 | 1092 |
| 760 | 983 | 1074 |
| 744 | 938 | 1048 |
| 697 | 925 | 1031 |
| 684 | 910 | 1015 |
| 642 | 904 | 1002 |
| 598 | 882 | 984 |
| 555 | 864 | 940 |
| 534 | 823 | 924 |
| 500 | 812 | 908 |
| 446 | 786 | 895 |
| 424 | 763 | 883 |
| 362 | 735 | 837 |
| 310 | 713 | 826 |
| 294 | 694 | 788 |
| 263 | 688 | 761 |
| 215 | 649 | 739 |
| 159 | 623 | 710 |
| 103 | 614 | 691 |
| 85 | 594 | 649 |
| | 580 | 620 |
| | 555 | 593 |
| | 512 | 581 |
| | 464 | 517 |
| | 445 | 469 |
| | 421 | 431 |
| | 394 | 394 |
| | 356 | 370 |
| | 345 | 347 |
| | 254 | 333 |
| | 218 | 283 |
| | 186 | 266 |
| | 147 | 242 |
| | 133 | 156 |
| | 98 | 122 |
| | 84 | 82 |

In einem weiteren Aspekt betrifft die vorliegende Erfindung eine Zusammensetzung, enthaltend die erfindungsgemäße Kristallform B in einer Gesamtmenge von mindestens 5 Gew.-%, wobei diese Gesamtmenge vorzugsweise mindestens 10, 15, 20, 25, 30, 35, 40, 45, 50, 55, 60, 65, 70, 75, 80, 85, 90, 95 Gew.-% oder mehr, beträgt, jeweils bezogen auf die in der Zusammensetzung enthaltene Gesamtmenge an Mononatrium-Salz von Foramsulfuron.

Eine solche erfindungsgemäße Zusammensetzung ist vorzugsweise dadurch gekennzeichnet, dass die Zusammensetzung die erfindungsgemäße Kristallform B in einer Gesamtmenge von mindestens 10 Gew.-%, vorzugsweise von 15 Gew.-% oder mehr, enthält, jeweils bezogen auf die in der Zusammensetzung enthaltene Gesamtmenge an Mononatrium-Salz von Foramsulfuron.

Eine bevorzugte erfindungsgemäße Zusammensetzung ist dadurch gekennzeichnet, dass sie bei 25 °C und 1013 mbar in fester Form vorliegt,
und/oder
der Gehalt an Mononatrium-Salz von Foramsulfuron bei 80 Gew.-% oder mehr liegt, vorzugsweise bei 85 Gew.-% oder mehr, bevorzugt bei 90 Gew.-% oder mehr, besonders bevorzugt bei 95 Gew.-% oder mehr, jeweils bezogen auf das Gesamtgewicht der Zusammensetzung.

Eine bevorzugte erfindungsgemäße Zusammensetzung ist dadurch gekennzeichnet, dass die Zusammensetzung die erfindungsgemäße Kristallform B in einer Gesamtmenge von mindestens 25 Gew.-%, vorzugsweise von 40 Gew.-% oder mehr, enthält, jeweils bezogen auf die in der Zusammensetzung enthaltene Gesamtmenge an Mononatrium-Salz von Foramsulfuron,
und
der Gehalt an Mononatrium-Salz von Foramsulfuron bei 80 Gew.-% oder mehr liegt, vorzugsweise bei 85 Gew.-% oder mehr, jeweils bezogen auf das Gesamtgewicht der Zusammensetzung.

Eine besonders bevorzugte erfindungsgemäße Zusammensetzung ist dadurch gekennzeichnet, dass die Zusammensetzung die erfindungsgemäße Kristallform B in einer Gesamtmenge von mindestens 50 Gew.-%, vorzugsweise von 60 Gew.-% oder mehr, enthält, jeweils bezogen auf die in der Zusammensetzung enthaltene Gesamtmenge an Mononatrium-Salz von Foramsulfuron,
und
der Gehalt an Mononatrium-Salz von Foramsulfuron bei 85 Gew.-% oder mehr liegt, vorzugsweise bei 90 Gew.-% oder mehr, jeweils bezogen auf das Gesamtgewicht der Zusammensetzung.

Eine ganz besonders bevorzugte erfindungsgemäße Zusammensetzung ist dadurch gekennzeichnet, dass die Zusammensetzung die erfindungsgemäße Kristallform B in einer Gesamtmenge von mindestens 70 Gew.-%, vorzugsweise von 80 Gew.-% oder mehr, enthält, jeweils bezogen auf die in der Zusammensetzung enthaltene Gesamtmenge an Mononatrium-Salz von Foramsulfuron,
und
der Gehalt an Mononatrium-Salz von Foramsulfuron bei 85 Gew.-% oder mehr liegt, vorzugsweise bei 90 Gew.-% oder mehr, jeweils bezogen auf das Gesamtgewicht der Zusammensetzung.

Eine ganz besonders bevorzugte erfindungsgemäße Zusammensetzung ist dadurch gekennzeichnet, dass die Zusammensetzung die erfindungsgemäße Kristallform B in einer Gesamtmenge von mindestens 80 Gew.-%, vorzugsweise von 85 Gew.-% oder mehr, enthält, jeweils bezogen auf die in der Zusammensetzung enthaltene Gesamtmenge an Mononatrium-Salz von Foramsulfuron, und
der Gehalt an Mononatrium-Salz von Foramsulfuron bei 90 Gew.-% oder mehr liegt, vorzugsweise bei 95 Gew.-% oder mehr, jeweils bezogen auf das Gesamtgewicht der Zusammensetzung.

Eine der am meisten besonders bevorzugten Ausgestaltung ist eine erfindungsgemäße Zusammensetzung dadurch gekennzeichnet, dass die Zusammensetzung die erfindungsgemäße Kristallform B in einer Gesamtmenge von mindestens 90 Gew.-%, vorzugsweise von 95 Gew.-% oder mehr, enthält, jeweils bezogen auf die in der Zusammensetzung enthaltene Gesamtmenge an Mononatrium-Salz von Foramsulfuron,
und
der Gehalt an Mononatrium-Salz von Foramsulfuron bei 90 Gew.-% oder mehr liegt, vorzugsweise bei 95 Gew.-% oder mehr, jeweils bezogen auf das Gesamtgewicht der Zusammensetzung.

In einem weiteren Aspekt betrifft die vorliegende Erfindung die Verwendung der erfindungsgemäßen Kristallform B oder einer erfindungsgemäßen Zusammensetzung wie vorstehend definiert zur Herstellung von agrochemischen Zubereitungen, vorzugsweise zur Herstellung von herbizid wirksamen agrochemischen Zubereitungen.

In einem weiteren Aspekt betrifft die vorliegende Erfindung das Mono-Methanol-Solvat des Mononatrium-Salzes von Foramsulfuron in kristalliner Form (Kristallform C), dadurch gekennzeichnet, dass das Röntgen-Pulverdiffraktogramm dieses Salzes bei Verwendung von Cu Kα-Strahlung bei 25 °C mindestens 3 der folgenden 2Θ (2 Theta)-Werte aufweist:

| 2Θ (2 Theta)-Werte in ° |
|---|
| 9.1 |
| 9.1 |
| 9.7 |
| 12.6 |
| 16.2 |
| 18.5 |
| 24.1 |
| 24.3 |
| 24.6 |

Bevorzugt weist das Röntgen-Pulverdiffraktogramm der erfindungsgemäßen Kristallform C bei Verwendung von Cu Kα-Strahlung bei 25 °C mindestens 4, vorzugsweise mindestens 6, bevorzugt mindestens 8, weiter bevorzugt sämtliche, der vorstehend genannten 2Θ (2 Theta)-Werte auf.

Weiter bevorzugt weist das Röntgen-Pulverdiffraktogramm der erfindungsgemäßen Kristallform C bei Verwendung von Cu Kα-Strahlung bei 25 °C zusätzlich mindestens zwei, vier, sechs, acht, zehn, zwölf oder mehr, vorzugsweise mindestens 15, 20, 23, 25, 27, 30, 33, 35, 38, 40, 42, 44, 46, 48, 50 oder mehr, bevorzugt sämtliche, der folgenden 2Θ (2 Theta)-Werte auf:

| |
|---|
| 12.0 |
| 14.8 |
| 15.7 |
| 16.3 |
| 17.8 |
| 18.0 |
| 18.2 |
| 19.0 |
| 19.5 |
| 19.9 |
| 20.1 |
| 20.9 |
| 21.1 |
| 21.7 |
| 22.1 |
| 22.6 |
| 23.5 |
| 23.8 |
| 24.5 |
| 25.1 |
| 25.6 |
| 25.8 |
| 26.5 |
| 26.8 |
| 27.1 |
| 28.8 |
| 29.2 |
| 29.7 |
| 30.0 |
| 30.5 |
| 30.8 |
| 31.3 |
| 31.7 |
| 31.9 |
| 32.0 |
| 33.1 |
| 33.2 |
| 33.5 |
| 34.2 |
| 34.4 |
| 34.9 |
| 35.4 |
| 35.8 |
| 36.2 |
| 36.6 |
| 37.2 |
| 37.7 |

Bevorzugt ist die erfindungsgemäße Kristallform C dadurch gekennzeichnet, dass ihr Raman-Spektrum mindestens folgende Banden aufweist:

| Bandenmaximum [cm⁻¹] |
|---|
| 3090 |
| 2943 |
| 2919 |
| 1593 |
| 1576 |
| 691 |
| 156 |
| 122 |

Bevorzugt weist das Raman-Spektrum der erfindungsgemäßen Kristallform C mindestens zehn der in Tabelle 4 angegebenen Banden auf, vorzugsweise 15, 20, 25, 30, 35, 40, 45, 50, 55, 60, 65 oder mehr, bevorzugt sämtliche, der in Tabelle 4 angegebenen Banden.

Bevorzugt entspricht das Raman-Spektrum der erfindungsgemäßen Kristallform C im Wesentlichen dem in Abbildung 6 wiedergegebenen Spektrum.

Ferner betrifft die vorliegende Erfindung eine Zusammensetzung, enthaltend die Kristallform C in einer Gesamtmenge von mindestens 5 Gew.-%, wobei diese Gesamtmenge vorzugsweise mindestens 10, 15, 20, 25, 30, 35, 40, 45, 50, 55, 60, 65, 70, 75, 80, 85, 90, 95 Gew.-% oder mehr, beträgt, jeweils bezogen auf die in der Zusammensetzung enthaltene Gesamtmenge an Mononatrium-Salz von Foramsulfuron.

Vorzugsweise betrifft die vorliegende Erfindung eine Zusammensetzung, enthaltend die Kristallform C (wie vorstehend definiert), wobei
die Zusammensetzung bei 25°C und 1013 mbar in fester Form vorliegt,
und/oder
der Gehalt an Mononatrium-Salz von Foramsulfuron bei 80 Gew.-% oder mehr liegt, wobei vorzugsweise der Gehalt an Mononatrium-Salz von Foramsulfuron bei 85 Gew.-% oder mehr liegt, bevorzugt bei 90 Gew.-% oder mehr, besonders bevorzugt bei 95 Gew.-% oder mehr, jeweils bezogen auf das Gesamtgewicht der Zusammensetzung.

Die erfindungsgemäße Kristallform B kann durch die im Folgenden beschriebenen Verfahren hergestellt werden.

Die Erfindung betrifft ferner ein Verfahren zur Herstellung der Kristallform B, umfassend den Schritt
(a) Suspendieren eines Mononatrium-Salzes von Foramsulfuron (vorzugsweise der Kristallform A), der Kristallform C, oder eines Gemisches dieser Substanzen, in einem Verdünnungsmittel oder Verdünnungsmittelgemisch,
wobei das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 20 Gew.-%, bevorzugt zu mindestens 30 Gew.-%, weiter bevorzugt zu mindestens 40 Gew.-%, noch weiter bevorzugt zu mindestens 50 Gew.-% daraus besteht.

Die erfindungsgemäße Kristallform B kann erhalten werden, wenn das in dem vorstehend beschriebenen Verfahren eingesetzte Verdünnungsmittel oder Verdünnungsmittelgemisch 20 Gew.-% oder mehr an Alkoholen mit 3 bis 6 C-Atomen und/oder Ketonen mit 3 bis 6 C-Atomen enthält.

Bevorzugt ist es, wenn das in dem vorstehend beschriebenen Verfahren eingesetzte Verdünnungsmittel oder Verdünnungsmittelgemisch 60 Gew.-% oder mehr an Alkoholen mit 3 bis 6 C-Atomen und/oder Ketonen mit 3 bis 6 C-Atomen enthält, da auf diese Weise besser filtrierbare Kristalle erhalten werden, was verfahrenstechnisch von erheblichem Vorteil ist.

Die Erfindung betrifft daher insbesondere ein Verfahren zur Herstellung der Kristallform B, umfassend den Schritt
(a) Suspendieren eines Mononatrium-Salzes von Foramsulfuron (vorzugsweise der Kristallform A), der Kristallform C, oder eines Gemisches dieser Substanzen, in einem Verdünnungsmittel oder Verdünnungsmittelgemisch,
wobei das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 60 Gew.-% daraus besteht.

Die Erfindung betrifft ferner ein Verfahren zur Herstellung der Kristallform B, umfassend den Schritt
(a) Suspendieren von Foramsulfuron in einem Verdünnungsmittel oder Verdünnungsmittelgemisch,
(b) Umsetzung von Foramsulfuron mit einer Natrium enthaltenden Base, vorzugsweise einer organischen Base, dabei bevorzugt Natriummethylat und/oder Natriumethylat,
wobei das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 20 Gew.-%, bevorzugt zu mindestens 30 Gew.-%, weiter bevorzugt zu mindestens 40 Gew.-%, noch weiter bevorzugt zu mindestens 50 Gew.-% daraus besteht.

Die Erfindung betrifft insbesondere ein Verfahren zur Herstellung der Kristallform B, umfassend den Schritt
(a) Suspendieren von Foramsulfuron in einem Verdünnungsmittel oder Verdünnungsmittelgemisch,
(b) Umsetzung von Foramsulfuron mit einer Natrium enthaltenden organischen Base, vorzugsweise Natriummethylat und/oder Natriumethylat,
wobei das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 60 Gew.-% daraus besteht.

Vorzugsweise wird dabei in Schritt (b) als Natrium enthaltenden organische Base Natriummethylat in Methanol und/oder Natriumethylat in Ethanol eingesetzt.

Die erfindungsgemäßen Verfahren zur Herstellung der Kristallform B werden vorzugsweise in der Weise ausgeführt, dass das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 60 Gew.-%, vorzugsweise zu 70 Gew.-% oder mehr, bevorzugt zu 80 Gew.-% oder mehr, weiter bevorzugt zu 90 Gew.-% oder mehr, besonders bevorzugt zu 95 Gew.-% oder mehr, daraus besteht.

Die erfindungsgemäßen Verfahren zur Herstellung der Kristallform B werden bevorzugt in der Weise ausgeführt, dass das in Schritt (a) verwendete Verdünnungsmittel oder Verdünnungsmittelgemisch ausgewählt ist aus der Gruppe bestehend aus 1 -Butanol, Aceton, Methylisobutylketon, und deren Mischungen, oder zu 60 Gew.-% oder mehr (vorzugsweise zu 70 Gew.-% oder mehr, bevorzugt zu 80 Gew.-% oder mehr, weiter bevorzugt zu 90 Gew.-% oder mehr, besonders bevorzugt zu 95 Gew.-% oder mehr) daraus besteht.

Die erfindungsgemäßen Verfahren zur Herstellung der Kristallform B werden bevorzugt bei Temperaturen von mindestens 0 °C durchgeführt. Vorzugsweise werden die erfindungsgemäßen Verfahren zur Herstellung der Kristallform B bei Temperaturen im Bereich von 0 bis 150 °C durchgeführt, bevorzugt bei Temperaturen im Bereich von 25 bis 130 °C.

Die erfindungsgemäßen Verfahren zur Herstellung der Kristallform B werden bevorzugt bei Temperaturen von mindestens 25 °C durchgeführt, vorzugsweise von mindestens 30 °C, bevorzugt von mindestens 40 °C. Vorzugsweise werden die erfindungsgemäßen Verfahren zur Herstellung der Kristallform B bei Temperaturen im Bereich von 40 bis 150 °C durchgeführt, bevorzugt bei Temperaturen im Bereich von 45 bis 130 °C, besonders bevorzugt im Bereich von 50 bis 120 °C.

In besonders bevorzugten erfindungsgemäßen Verfahren zur Herstellung der Kristallform B wird Aceton in Schritt (a) als ein oder als das Verdünnungsmittel verwendet. In diesem Fall werden die erfindungsgemäßen Verfahren zur Herstellung vorzugsweise bei Temperaturen im Bereich von 0 bis 65 °C durchgeführt, bevorzugt im Bereich von 25 bis 56 °C.

In weiter besonders bevorzugten erfindungsgemäßen Verfahren zur Herstellung der Kristallform B wird Aceton in Schritt (a) als ein oder als das Verdünnungsmittel verwendet. In diesem Fall werden die erfindungsgemäßen Verfahren zur Herstellung vorzugsweise bei Temperaturen im Bereich von 40 bis 65 °C durchgeführt, bevorzugt im Bereich von 45 bis 56 °C, besonders bevorzugt im Bereich von 50 bis 56 °C.

Die erfindungsgemäßen Verfahren zur Herstellung der Kristallform B werden bevorzugt in der Weise ausgeführt, dass das Gewichtsverhältnis der Gesamtmenge an Foramsulfuron, Kristallform A, und Kristallform C zu der Gesamtmenge des in Schritt (a) eingesetzten Verdünnungsmittels oder oder Verdünnungsmittelgemisches kleiner als 2 : 1 ist. Vorzugsweise liegt das Gewichtsverhältnis der Gesamtmenge an Foramsulfuron, Mononatrium-Salzen von Foramsulfuron (vorzugsweise der Kristallform A), und der Kristallform C zu der Gesamtmenge des in Schritt (a) eingesetzten Verdünnungsmittels oder Verdünnungsmittelgemisches im Bereich von 3 : 1 bis 1 : 20, bevorzugt im Bereich von 2 : 1 bis 1 : 15, weiter bevorzugt im Bereich von 1 : 1 bis 1 : 10. In vielen Fällen liegt dieses Gewichtsverhältnis vorzugsweise im Bereich von 2 : 3 bis 1 : 8, und insbesondere im Bereich von 1 : 2 bis 1 : 5.

Das erfindungsgemäße Verfahren zur Herstellung der erfindungsgemäßen Kristallform B umfasst vorzugsweise weitere Schritte, dabei vorzugsweise ein Abkühlen des die Kristallform B enthaltenden resultierenden Gemisches. Das Abkühlen erfolgt dabei vorzugsweise über einen Zeitraum von mindestens 15 Minuten, bevorzugt von mindestens 30 Minuten.

Das Abkühlen erfolgt dabei weiter bevorzugt über einen Zeitraum von mindestens 60 Minuten, noch weiter bevorzugt von mindestens 120 Minuten, und insbesondere bevorzugt von mindestens 180 Minuten.

Das erfindungsgemäße Verfahren zur Herstellung der erfindungsgemäßen Kristallform B umfasst vorzugsweise weitere Schritte, dabei vorzugsweise

Filtration des die Kristallform B enthaltenden resultierenden Gemisches, und/oder
Isolierung der Kristallform B, und/oder
Trocknung Kristallform B.

Es ist im Rahmen des erfindungsgemäßen Verfahrens zur Herstellung der erfindungsgemäßen Kristallform B bevorzugt, wenn das eingesetzte Ausgangsmaterial, vorzugsweise die Kristallform A und/oder die Kristallform C, im Verdünnungsmittel höchstens geringfügig gelöst wird, und insbesondere - im Gegensatz zu Umkristallisationsprozessen - nicht vollständig aufgelöst wird, sondern eine fest-fest Umwandlung des Ausgangsmaterials in die Kristallform B, d.h. eine Umwandlung fester Stoffe, erfolgt. Beschrieben wird ferner eine Mischung, umfassend
(i) Mononatrium-Salz von Foramsulfuron und/oder ein Solvat davon, vorzugsweise umfassend Kristallform A, Kristallform B, Kristallform C, oder eine Mischung umfassend zwei oder sämtliche dieser Kristallformen, und
(ii) ein Verdünnungsmittel oder Verdünnungsmittelgemisch, ausgewählt aus der Gruppe bestehend aus Alkoholen mit 3 bis 6 C-Atomen, dabei vorzugsweise primären Alkoholen mit 3 bis 6 C-Atomen, und Kctoncn mit 3 bis 6 C-Atomen, und deren Mischungen, oder zu mindestens 20 Gcw.-%, bevorzugt zu mindestens 30 Gew.-%, weiter bevorzugt zu mindestens 40 Gew.-%, noch weiter bevorzugt zu mindestens 50 Gew.-% daraus besteht.

Beschrieben wird insbesondere eine Mischung, umfassend
(i) Mononatrium-Salz von Foramsulfuron und/oder ein Solvat davon, vorzugsweise umfassend Kristallform A, Kristallform B, Kristallform C, oder eine Mischung umfassend zwei oder sämtliche dieser Kristallformen, und
(ii) ein Verdünnungsmittel oder Verdünnungsmittelgemisch, ausgewählt aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 60 Gew.-% daraus besteht, und vorzugsweise zu 70 Gew.-% oder mehr, bevorzugt zu 80 Gew.-% oder mehr, weiter bevorzugt zu 90 Gew.-% oder mehr, besonders bevorzugt zu 95 Gew.-% oder mehr daraus besteht.

Vorzugsweise ist das Verdünnungsmittel oder Verdünnungsmittelgemisch einer solchen erfindungsgemäßen Mischung ausgewählt aus der Gruppe bestehend aus 1-Butanol, Aceton, Methylisobutylketon, und deren Mischungen, oder es besteht mindestens zu 60 Gew.-% (vorzugsweise zu 70 Gew.-% oder mehr, bevorzugt zu 80 Gew.-% oder mehr, weiter bevorzugt zu 90 Gew.-% oder mehr, besonders bevorzugt zu 95 Gew.-% oder mehr) daraus.

Das Gewichtsverhältnis der Gesamtmenge des Bestandteils (a) Mononatrium-Salzes von Foramsulfuron zu der Gesamtmenge des Bestandteils (b) Verdünnungsmittel oder Verdünnungsmittelgemisch in einer erfindungsgemäßen Mischung entspricht vorzugsweise den vorstehend (als bevorzugt) angegebenen Gewichtsverhältnissen, jeweils bezogen auf das Gesamtgewicht der Mischung, die im Zusammenhang mit dem Herstellungsverfahren der erfindungsgemäßen Kristallform B angegeben sind.

Die erfindungsgemäße Kristallform B kann als solche oder in Form von agrochemischen Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der nachstehend genannten Formulierungen hergestellt werden, wobei die physikalischen Eigenschaften und Stabilitäten der zu kombinierenden Wirkstoffe zu berücksichtigen sind. Die erfindungsgemäße Kristallform B kann auf verschiedene Art formuliert werden, wobei in der Regel im Pestizidbereich übliche Formulierhilfsstoffe eingesetzt werden und/oder weitere (agrochemische) Wirkstoffe.

Die Erfindung betrifft daher in einem weiteren Aspekt eine agrochemische Zubereitung, enthaltend
(a) eine herbizid wirksame Menge der Kristallform B,
   und ein oder mehrere weitere Bestandteile ausgewählt aus der Gruppe bestehend aus den nachfolgenden Bestandteilen (b-i), (b-ii) und (b-iii):
(b-i) im Pestizidbereich übliche Formulierhilfsstoffe,
(b-ii) weitere agrochemische Wirkstoffe,
   und
(b-iii) der Kristallform C.

Vorzugsweise enthält eine erfindungsgemäße Formulierung als Bestandteil (b-ii) vorzugsweise ein oder mehrere Safener (dabei vorzugsweise Isoxadifen, Isoxadifen-Ethyl, und/oder Cyprosulfamid) und/oder ein oder mehrere weitere herbizide Wirkstoffe.

Die weiteren herbiziden Wirkstoffe sind dabei vorzugsweise ausgewählt aus den im "The Pesticide Manual", 16th edition, The British Crop Protection Council and the Royal Society of Chemistry, 2012 genannten herbiziden Wirkstoffen. Die bevorzugten weiteren herbiziden Wirkstoffe sind dabei die in EP 0 790 771 A1 oder EP 1 104 239 A1 genannten herbiziden Wirkstoffe, insbesondere weitere ALS-Inhibitoren (Acetolactat-Synthase-Inhibitoren), dabei wiederum bevorzugt Thiencarbazone-methyl und dessen Salze und/oder weitere Sulfonylharnstoff-Herbizide, dabei wiederum bevorzugt Amidosulfuron, Mesosulfuron, Mesosulfuron-methyl, Iodosulfuron, Iodosulfuron-methyl, und deren jeweiligen Salze, wie beispielsweise Iodosulfuron-Natrium oder Iodosulfuron-methyl-Natrium (zu den besonderen Vorteilen bezüglich der (Stabilitäts)Verbesserung in Kombination mit anderen Sulfonylharnstoff-Herbiziden wurde eingangs bereits ausgeführt).

Daneben enthalten die genannten agrochemischen Zubereitungen (Formulierungen) gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Als Formulierungsmöglichkeiten für erfindungsgemäße agrochemische Zubereitungen enthaltend die erfindungsgemäße Kristallform B kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Ferner wurde gefunden, dass in bestimmten erfindungsgemäßen agrochemischen Zubereitungen, beispielsweise einigen Öl-Dispersionen (OD) es von Vorteil ist, eine Mischung der erfindungsgemäßen Kristallform B und der erfindungsgemäßen Kristallform C ein zusetzen. Solche erfindungsgemäßen agrochemischen Zubereitungen sind dabei vorzugsweise an Formulierungen angelehnt, wie sie beispielsweise in US 2002/0016263 A1 oder US 2005/0032647 A1 beschrieben sind.

Solche agrochemischen Zubereitungen enthaltend eine Mischung der erfindungsgemäßen Kristallform B und der erfindungsgemäßen Kristallform C zeigten dabei gegenüber ansonsten identischen Formulierungen, die nur Kristallform B enthielten, weiter verbesserte Eigenschaften, beispielsweise eine weiter verbesserte Lagerstabilität, dabei insbesondere hinsichtlich der Fließfähigkeit und Viskosität der Formulierung.

In solchen erfindungsgemäßen agrochemischen Zubereitungen liegt das Gewichtsverhältnis der Gesamtmenge an Kristallform B zu der Gesamtmenge an Kristallform C vorzugsweise im Bereich von 20 : 1 bis 1 : 10, bevorzugt im Bereich von 10 : 1 bis 1 : 5, weiter bevorzugt im Bereich von 10 : 1 bis 1 : 3, und besonders bevorzugt im Bereich von 5 : 1 bis 1 : 1, jeweils bezogen auf das Gesamtgewicht der erfindungsgemäßen agrochemischen Zubereitung.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff ausser einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Bevorzugte erfindungsgemäße agrochemische Zubereitungen sind solche, in denen die erfindungsgemäße Kristallform B in fester Form vorliegt. Es handelt sich dabei beispielsweise um Granulate, verkapselte Granulate, Tabletten, wasserdispergierbare Granulate, wasserdispergierbare Tabletten, wasserdispergierbare Pulver, Staub-Formulierungen, Formulierungen, in denen der Wirkstoff in dispergierter Form vorliegt wie z.B.: Suspensionskonzentrate (SC), Öl basierte Suspensionskonzentrate, Suspo-emulsionen, oder Suspensionskonzentrate.

Besonders bevorzugte erfindungsgemäße agrochemische Zubereitungen enthaltend die erfindungsgemäße Kristallform B sind solche, wobei die agrochemischen Zubereitung in einer Form vorliegt ausgewählt aus der Gruppe bestehend aus Spritzpulver (WP), wasserlösliche Pulver (SP), Suspensionskonzentrate (SC), Öl-Dispersionen (OD) Kapselsuspensionen (CS), Stäubemittel (DP), Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die erfindungsgemäßen agrochemischen Zubereitungen enthalten in der Regel 0,1 bis 99 Gew.-%, insbesondere 0,1 bis 95 Gew.-%, der erfindungsgemäßen Kristallform B, bezogen auf das Gesamtgewicht der agrochemischen Zubereitung, wobei die Gesamtmenge der erfindungsgemäßen Kristallform B je nach Formulierungen variieren kann.

In Spritzpulvern beträgt die Gesamtmenge an erfindungsgemäßer Kristallform B typischerweise 10 bis 90 Gew.-%, der Rest zu 100 Gew.-% besteht aus üblichen Formulierungsbestandteilen.

Bei emulgierbaren Konzentraten kann die Gesamtmenge an erfindungsgemäßer Kristallform B etwa 1 bis 90, vorzugsweise 5 bis 80 Gew.-% betragen. Staubförmige Formulierungen enthalten regelmäßig eine Gesamtmenge an erfindungsgemäßer Kristallform B von 1 bis 30 Gew.-%, vorzugsweise von 5 bis 20 Gew.-%. Bei in Wasser dispergierbaren Granulaten liegt die Gesamtmenge an erfindungsgemäßer Kristallform B beispielsweise im Bereich von 1 und 95 Gew.-%, im Bereich von 10 und 80 Gew.-%, bezogen auf das Gesamtgewicht der agrochemischen Zubereitung.

Gegenstand der Erfindung ist daher auch ein Verfahren zur Bekämpfung von Schadpflanzen und/oder zur Wachstumsregulierung von Pflanzen, dadurch gekennzeichnet, dass eine wirksame Menge
- der Kristallform B,
- einer Zusammensetzung enthaltend die Kristallform B, wie oben definiert,
   oder
- einer erfindungsgemäßen agrochemischen Zubereitung, wie oben definiert,
auf die Pflanzen, Pflanzensamen, den Boden, in dem oder auf dem die Pflanzen wachsen, oder die Anbaufläche appliziert wird.

Ferner wurde gefunden, dass die erfindungsgemäße Kristallform B, erfindungsgemäße Mischungen, erfindungsgemäße Zusammensetzungen, und erfindungsgemäße Formulierungen, jeweils wie im Rahmen der vorliegenden Erfindung definiert, zur Bekämpfung von Schadpflanzen oder zur Wachstumsregulierung von Pflanzen geeignet sind. Die Bekämpfung von Schadpflanzen oder zur Wachstumsregulierung von Pflanzen erfolgt vorteilhafterweise in bestimmten Kulturpflanzen. Bevorzugte Kulturpflanzen sind dabei insbesondere Raps, Soja, Baumwolle, Zuckerrübe und Süßgräser, dabei insbesondere Mais, Gerste, Weizen, Roggen, Hafer, Triticale, Hirsen, Reis, wobei Mais dabei besonders bevorzugt ist. In einer bevorzugten Ausgestaltung sind die Kulturpflanzen, insbesondere die als bevorzugt angegebenen Kulturpflanzen, transgene Pflanzen.

Daneben ist auch eine Anwendung auf Rasenflächen, wie beispielweise auf Golfplätzen, möglich.

### Beispiele:

Die nachfolgenden Beispiele verdeutlichen die Erfindung. Sofern nicht anders angegeben, beziehen sich alle Angaben auf das Gewicht.

Das Symbol ">" bedeutet "größer als und das Symbol und "< " bedeutet "kleiner als".

### Beispiel 1: Herstellung der Kristallform A

1000 g N-[(4,6-Dimethoxypyridin-2-yl)-aminocarbonyl]-2-dimethylaminocarbonyl-5-formylamino-benzolsulfonamid wurden in 4750 g 5 °C kaltem MeOH (6000 mL) vorgelegt.

Hierzu wurden 32 g Impfkristalle der Kristallform A (gemäß EP 0 757 679 A1) zugegeben und die Mischung in 20 Minuten auf 0 °C abgekühlt. Nach Erreichen einer Temperatur von 0 °C wurden 418 g (440 mL) 30 % ige Natriummethylat-Lösung innerhalb von 2 Stunden zudosiert, so dass eine Innentemperatur von 5 °C nicht überschritten wurde.

Die resultierende sehr pastöse Mischung wurde anschließend auf zwei kühlbaren 4 L Fritten verteilt und abgesaugt. Nach etwa 2 Stunden wurde die Filtration beendet und auf jeder Fritte mit 500 mL nachgewaschen.

Insgesamt wurden 2220 g des Feuchtproduktes erhalten, welche anschließend bei 65 °C bei einem Druck < 100 mbar getrocknet wurden. Die Trocknung dauert insgesamt 44 h. Bei einem Methanolgehalt von 1,5 Gew.-% Rest wurde die Trocknung beendet. Es wurden 1012 g eines 97 % igen Produktes erhalten.

### Beispiel 2: Variation der Temperatur bei der Herstellung der Kristallform A in Methanol

In separaten Behältnissen wurden je 20 g N-[(4,6-Dimethoxypyridin-2-yl)-aminocarbonyl]-2-dimethylaminocarbonyl-5-formylamino-benzolsulfonamid in je 100 g MeOH vorgelegt.

Es wurde jeweils 1 g an Impfkristallen der Kristallform A zugegeben und bei der jeweils angegebenen Temperatur über einen Zeitraum von 2 Stunden ein Äquivalent NaOMe als 30 % ige methanolische Lösung zugegeben. Die Nachreaktionszeit betrug jeweils 3 Stunden.

Zur Isolierung der jeweiligen Produkte wurde die jeweilige Mischung auf 20 °C erwärmt bzw. abgekühlt und anschließend filtriert. Nach der Trocknung bei 65 °C bei einem Druck < 100 mbar wurde der Restgehalt an Methanol in jedem Produkt bestimmt.

| Versuch-Nr.. | T in °C | Filtrationszeit in s | MeOH-Gehalt in Gew.-% |
|---|---|---|---|
| 1 | 0 | 150 | 0,53 |
| 2 | 15 | 50 | 1,49 |
| 3 | 35 | 4 | 2,83 |
| 4 | 50 | 5 | 5,19 |

### Beispiel 3: Lagerung der methanolfeuchten Kristallform A und deren Umwandlung in Kristallform C

In separaten Behältnissen wurden je 5 g der Kristallform A mit je 20 g MeOH verrührt und anschließend abfiltriert.

Bei 20 °C, 30 °C und 35 °C wurden die angefeuchteten Produkte eine Woche gelagert und täglich eine Probe genommen. Diese Proben wurden bei 65 °C und 100 mbar getrocknet und danach der jeweilige Methanolgehalt ermittelt.

Bereits nach 2 Tagen (35 °C), 3 Tagen (30 °C) und 7 Tagen (20 °C) hatte sich die Kristallform A praktisch vollständig in die Kristallform C umgewandelt (Gehalt an MeOH etwa 6,4 Gew.-%).

### Beispiel 4: Herstellung der Kristallform B aus der Kristallform A

1000 g N-[(4,6-Dimethoxypyridin-2-yl)-aminocarbonyl]-2-dimethylaminocarbonyl-5-formylaminobenzolsulfonamid Na-Salz (Kristallform A) wurden in 3000 g Aceton suspendiert und 2 Stunden unter Rückfluss erhitzt. Nach dem Abkühlen auf 20 °C wurde die Suspension filtriert. Die Filtration dauerte 60 Minuten.

Das so erhaltene Feuchtprodukt wurde anschließend bei 70 °C und einem Druck < 100 mbar über Nacht getrocknet. Es wurden 9230 g eines 98,6 % igen Produktes erhalten. Der MeOH Gehalt in diesem Produkt lag bei < 0,1 Gew.-%, der Acetongehalt bei < 0,05 Gew-%.

### Beispiel 5: Herstellung der Kristallform B aus Kristallform C

100 g N-[(4,6-Dimethoxypyridin-2-yl)-aminocarbonyl]-2-dimethylaminocarbonyl-5-formylaminobenzolsulfonamid Na-Salz (Kristallform C; MeOH-Gehalt etwa 6,4 %) wurden in 300 g Aceton suspendiert und 2 Stunden unter Rückfluss erhitzt. Nach dem Abkühlen auf 20 °C wurde die Suspension filtriert. Die Filtration dauerte wenige Sekunden.

Das Feuchtprodukt wurde bei 70 °C und einem Druck von < 100 mbar) über Nacht getrocknet.

Es wurden 92 g eines 99,2 % igen Produktes erhalten. Der Gehalt an Methanol in diesem Produkt lag unterhalb der Nachweisgrenze, der Gehalt an Aceton lag bei 0,026 Gew.%.

### Beispiel 6: Herstellung der Kristallform B aus Foramsulfuron und Natriummethylat

100 g N-[(4,6-Dimethoxypyridin-2-yl)-aminocarbonyl]-2-dimethylaminocarbonyl-5-formylaminobenzolsulfonamid wurden in 300 g Aceton suspendiert und bei 40 °C innerhalb von einer Stunde mit einem Äquivalent an NaOMe als 30 % ige methanolische Lösung (40,21 g) versetzt . Anschließend wurde die resultierende Mischung 2 Stunden unter Rückfluss erhitzt. Nach dem Abkühlen auf 20 °C wurde die Suspension filtriert. Die Filtration dauerte ca. 30 Sekunden.

Das Feuchtprodukt wurde bei 70 °C und einem Druck von < 100 mbar über Nacht getrocknet.

Es wurden 104 g eines 98,4 % igen Produktes erhalten. Der Gehalt an Methanol in dem erhaltenen Produkt lag bei 0,006 Gew.-%, der Gehalt an der Aceton lag bei 0,048 Gew.-%.

### Beispiel 7: Herstellung der Kristallform B aus Foramsulfuron und Natriumethylat

100 g N-[(4,6-Dimethoxypyridin-2-yl)-aminocarbonyl]-2-dimethylaminocarbonyl-5-formylaminobenzolsulfonamid wurden in 400 g Aceton suspendiert und unter Rückfluss innerhalb von einer Stunde mit einem Äquivalent an NaOEt als 21 % ige ethanolische Lösung (73,1 g) versetzt . Anschließend wurde noch 2 Stunden unter Rückfluss erhitzt. Nach dem Abkühlen auf 20 °C wurde die Suspension filtriert. Die Filtration dauerte ca. 60 Sekunden.

Das Feuchtprodukt wurde bei 70 °C und einem Druck von < 100 mbar) über Nacht getrocknet.

Es wurden 102 g eines 98,6 % igen Produktes erhalten. Der Gehalt an Ethanol in dem erhaltenen Produkt lag bei 0,0105 Gew.-%, der Gehalt an der Aceton lag bei 0,024 Gew.-%.

### Beispiel 8: Stabilitätsprüfung der Kristallform B in Methanol

10 g der Kristallform B (Gehalt an MeOH < 0,01 %) wurden in 100 g Methanol suspendiert und 7 Tage bei 30 °C gerührt. Dann wurde filtriert und anschließend bei 70 °C und einem Druck < 100 mbar) über Nacht getrocknet.

Es wurden 9,1 g eines 99,4 % igen Produktes erhalten. Der Gehalt an Methanol in dem erhaltenen Produkt lag bei 0,004 Gew.-%, der Gehalt an Aceton lag bei 0,0003 Gew.-%.

### Beispiel 9: Untersuchung der chemischen Lagerstabilität der Kristallformen A und B

Die Kristallformen A und B wurden jeweils separat in eine ansonsten identische Formulierung in Form von Öl-Dispersionen (OD) eingearbeitet und unter denselben Bedingungen gelagert.

Die Öl-Dispersionen wurden unter den folgenden Lagerungsbedingungen gelagert und hinsichtlich des chemischen Abbaus der Kristallform A bzw. B analysiert:
Lagerung über zwei Wochen bei 40 °C (nachfolgend als 2W 40°C angegeben)
Lagerung über vier Wochen bei 40 °C (nachfolgend als 4W 40°C angegeben)
Lagerung über zwei Wochen bei 54 °C (nachfolgend als 2W 54°C angegeben)

Auf diese Weise wird auch eine Lagerung über einen längeren Zeitraum bei entsprechend niedrigerer Temperatur simuliert.

Die Kristallformen wurden in drei verschiedene Formulierungen in Form von Öl-Dispersionen (OD) eingearbeitet, wobei diese Formulierungen neben den im Folgenden angegebenen agrochemischen Wirkstoffen übliche Formulierungsbestandteile enthielten wie Emulgatoren, Verdicker, und Verdünnungsmittel sowie teilweise pH-Stellmittel und/oder Entschäumer.

Formulierung OD1 enthielt neben 3,05 Gew.-% an Kristallform A oder B als weitere Wirkstoffe 0,1 Gew.-% Iodosulfuron-Methyl-Natrium (ein Sulfonylharnstoff-Herbizid) und 2,91 Gew.% Isoxadifen-Ethyl (ein Safener).

Formulierung OD2 enthielt neben 3,21 Gew.-% an Kristallform A oder B als weitere Wirkstoffe 1,02 Gew.-% Thiencarbazone-Methyl und 1,53 Gew.% Cyprosulfamid (ein Safener).

Formulierung OD3 enthielt neben 3,21 Gew.-% an Kristallform A oder B als weitere Wirkstoffe 0,11 Gew.-% Iodosulfuron-Methyl-Natrium (ein Sulfonylharnstoff-Herbizid), 1,02 Gew.-% Thiencarbazone-Methyl und 1,53 Gew.% Cyprosulfamid (ein Safener).

**Tabelle 9-1: Chemische Stabilität der Kristallformen A und B in Formulierung OD1**

| Kristallform | Abbau in % | |
|---|---|---|
| | 4W 40°C | 2W 54°C |
| Kristallform A | 3,4 | 7,7 |
| Kristallform B | 1,5 | 3,4 |

**Tabelle 9-2: Chemische Stabilität der Kristallformen A und B in Formulierung OD2**

| Kristallform | Abbau in % | |
|---|---|---|
| | 2W 40°C | 2W 54°C |
| Kristallform A | 1,8 | 8,6 |
| Kristallform B | 0,6 | 1,8 |

**Tabelle 9-3: Chemische Stabilität der Kristallformen A und B in Formulierung OD3**

| Kristallform | Abbau in % | |
|---|---|---|
| | 4W 40°C | 2W 54°C |
| Kristallform A | 2,1 | 4,8 |
| Kristallform B | 0,3 | 1,4 |

### Beispiel 10: Herstellung der Kristallform B aus Foramsulfuron und Natriummethylat

100 g N-[(4,6-Dimethoxypyridin-2-yl)-aminocarbonyl]-2-dimethylaminocarbonyl-5-formylaminobenzolsulfonamid wurden in 300 g einer Mischung aus Aceton/Methanol (80:20 Gewichtsteile) suspendiert und bei 30 °C innerhalb von zwei Stunden mit einem Äquivalent an NaOMe als 30 % ige methanolische Lösung versetzt. Anschließend wurde noch 2 Stunden bei 30 °C nachgerührt. Nach dem Abkühlen auf 20 °C wurde die Suspension filtriert. Die Filtration dauerte 50 Sekunden.

Das Feuchtprodukt wurde bei 70 °C und einem Druck von < 100 mbar über Nacht getrocknet.

Es wurden 102 g eines 98,6 % igen Produktes erhalten. Der Gehalt an Methanol in dem erhaltenen Produkt lag bei 0,03 Gew.-%, der Gehalt an der Aceton lag bei 0,014 Gew.-%.

### Beispiel 11: Herstellung der Kristallform B aus Foramsulfuron und Natriummethylat

100 g N-[(4,6-Dimethoxypyridin-2-yl)-aminocarbonyl]-2-dimethylaminocarbonyl-5-formylaminobenzolsulfonamid wurden in einer Mischung aus 150 g Aceton und 100 g Methanol suspendiert und auf 30 °C erwärmt. Hierzu wurden innerhalb von zwei Stunden 38,6 g an NaOMe als 30 % ige methanolische Lösung gleichmäßig zudosiert. Anschließend wurde eine Stunde bei 30 °C nachgerührt. Dann wurde die Temperatur auf 40°C erhöht, und nach einer Stunde bei dieser Temperatur eine weitere Stunde bei 45°C.

Nach dem Abkühlen auf 20 °C wurde die Suspension filtriert, und der Filterkuchen mit 100 g Aceton gewaschen.

Das Feuchtprodukt wurde bei 70 °C und einem Druck von < 100 mbar über Nacht getrocknet.

Der Gehalt an Methanol in dem erhaltenen Produkt lag bei 0,10 Gew.-%, der Gehalt an der Aceton lag bei 0,13 Gew.-%.

### Beispiel 12: Sorptions-/Desorptionskurve der Kristallformen B

Es wurden isotherm bei 25°C Sorptions- und Desorptionskurven der Kristallform B gemessen.

Die Sorptionskurve gibt die Wasseraufnahme bei steigender relativer Luftfeuchte an, die Desorptionskurve das Trocknungsverhalten bei Feuchtereduktion.

| Relative Luftfeuchtigkeit | Sorption (Gew.-%) | Desorption (Gew.-%) |
|---|---|---|
| 10,0% | 0,100 | 0,846 |
| 20,0% | 0,201 | 1,227 |
| 30,0% | 0,313 | 1,495 |
| 40,0% | 0,444 | 1,760 |
| 50,0% | 0,566 | 2,086 |
| 60,0% | 0,684 | 2,269 |
| 70,0% | 0,864 | 2,485 |
| 80,0% | 1,085 | 3,016 |
| 90,0% | 2,335 | 5,151 |

Das Untersuchungsergebnis zeigt, dass Kristallform B nicht hygroskopisch ist.

### Beispiel 13: Untersuchung der Hygroskopie der Kristallformen A, B und C

Je 100 mg der Kristallformen A, B und C wurden separat in kleine Schnappdeckelgläser gefüllt. Diese drei Schnappdeckelgläser wurden unverschlossen in ein Becherglas gestellt, dessen Boden mit einer geringen Menge an Wasser bedeckt war. Das Becherglas wurde anschließend mit einer dehnbaren Verschlussfolie verschlossen und eine Woche lang bei 25 °C gelagert. Nach dieser Lagerung wurde der Wassergehalt der jeweiligen Probe per Karl-Fischer-Titration bestimmt. Der Wassergehalt der Probe der Kristallform B lag bei 0,4 Gew.-%, der Wassergehalt der Proben der Kristallformen A und C lag bei 15,7 bzw. 21,3 Gew.-%.

Die Proben der Kristallformen A und C waren hygroskopisch und nach der Lagerung sehr zäh und klebrig, wohingegen die Probe der Kristallform B weiterhin kristallin und pulverig war.

## Patentansprüche

1. Mononatrium-Salz von Foramsulfuron in kristalliner Form, **dadurch gekennzeichnet, dass** das Röntgen-Pulverdiffraktogramm dieses Salzes bei Verwendung von Cu Kα-Strahlung bei 25°C mindestens 3 der folgenden 2Θ (2 Theta)-Werte aufweist:
| 2Θ (2 Theta)-Werte in ° |
|---|
| 6.2 |
| 6.4 |
| 14.4 |
| 14.6 |
| 14.7 |
| 18.4 |
| 19.2 |
| 20.1 |
| 23.2 |
| 24.7 |

2. Mononatrium-Salz von Foramsulfuron gemäß Anspruch 1, **dadurch gekennzeichnet, dass** das Röntgen-Pulverdiffraktogramm dieses Salzes bei Verwendung von Cu Kα-Strahlung bei 25 °C mindestens 4, vorzugsweise mindestens 6, bevorzugt mindestens 8, weiter bevorzugt sämtliche, der in Anspruch 1 genannten 2Θ (2 Theta)-Werte aufweist.

3. Mononatrium-Salz von Foramsulfuron gemäß Anspruch 1, dadurch dass das Röntgen-Pulverdiffraktogramm dieses Salzes bei Verwendung von Cu Kα-Strahlung bei 25 °C zusätzlich mindestens zwei, vier, sechs, acht, zehn, zwölf oder mehr der folgenden 2Θ (2 Theta)-Werte aufweist:
| |
|---|
| 10.3 |
| 10.5 |
| 11.1 |
| 11.3 |
| 11.4 |
| 12.0 |
| 12.1 |
| 12.4 |
| 12.6 |
| 12.9 |
| 13.6 |
| 14.0 |
| 15.0 |
| 15.1 |
| 15.4 |
| 16.0 |
| 16.2 |
| 17.0 |
| 17.2 |
| 18.2 |
| 18.8 |
| 20.3 |
| 20.6 |
| 20.8 |
| 21.2 |
| 21.6 |
| 22.7 |
| 23.7 |
| 24.6 |
| 25.4 |
| 25.9 |
| 26.2 |
| 26.9 |
| 27.1 |
| 27.7 |
| 28.3 |
| 29.0 |
| 30.2 |
| 30.5 |
| 30.7 |
| 31.0 |
| 31.2 |
| 31.9 |
| 32.5 |
| 32.6 |
| 33.7 |
| 34.8 |
| 35.6 |
| 35.9 |
| 36.2 |
| 36.4 |
| 36.9 |
| 37.4 |

4. Mononatrium-Salz von Foramsulfuron gemäß einem der Ansprüche 1 bis 3, **dadurch gekennzeichnet, dass** sein Raman-Spektrum mindestens folgende Banden aufweist:
| Bandenmaximum [cm⁻¹] |
|---|
| 2959 |
| 2915 |
| 1686 |
| 1587 |
| 1526 |
| 694 |
| 345 |
| 133 |

5. Zusammensetzung, enthaltend das Mononatrium-Salz von Foramsulfuron gemäß einem der Ansprüche 1 bis 4 in einer Gesamtmenge von mindestens 5 Gew.-%, bezogen auf die in der Zusammensetzung enthaltene Gesamtmenge an Mononatrium-Salz von Foramsulfuron.

6. Zusammensetzung nach Anspruch 5, wobei
die Zusammensetzung bei 25 °C und 1013 mbar in fester Form vorliegt,
und/oder
der Gehalt an Mononatrium-Salz von Foramsulfuron bei 80 Gew.-% oder mehr liegt, bezogen auf das Gesamtgewicht der Zusammensetzung.

7. Verwendung des Mononatrium-Salzes von Foramsulfuron gemäß einem der Ansprüche 1 bis 4 oder einer Zusammensetzung nach Anspruch 5 oder 6 zur Herstellung von agrochemischen Zubereitungen.

8. Mono-Methanol-Solvat des Mononatrium-Salzes von Foramsulfuron in kristalliner Form, **dadurch gekennzeichnet, dass** das Röntgen-Pulverdiffraktogramm dieses Salzes bei Verwendung von Cu Kα-Strahlung bei 25 °C mindestens 3 der folgenden 2Θ (2 Theta)-Werte aufweist:
| 2Θ (2 Theta)-Werte in ° |
|---|
| 9.1 |
| 9.1 |
| 9.7 |
| 12.6 |
| 16.2 |
| 18.5 |
| 24.1 |
| 24.3 |
| 24.6 |

9. Verfahren zur Herstellung des Mononatrium-Salzes von Foramsulfuron in kristalliner Form gemäß einem oder mehreren der Ansprüche 1 bis 4, umfassend den Schritt
(a) Suspendieren eines Mononatrium-Salzes von Foramsulfuron, des Mono-Methanol-Solvates des Mononatrium-Salzes von Foramsulfuron nach Anspruch 8, oder eines Gemisches dieser beiden Kristallformen, in einem Verdünnungsmittel oder Verdünnungsmittelgemisch,
wobei das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 20 Gew.-% daraus besteht.

10. Verfahren zur Herstellung des Mononatrium-Salzes von Foramsulfuron in kristalliner Form gemäß einem der Ansprüche 1 bis 4, umfassend den Schritt
(a) Suspendieren von Foramsulfuron in einem Verdünnungsmittel oder Verdünnungsmittelgemisch,
(b) Umsetzung von Foramsulfuron mit einer Natrium enthaltenden Base, vorzugsweise einer organischen Base, dabei bevorzugt Natriummethylat und/oder Natriumethylat,
wobei das in Schritt (a) verwendete Verdünnungsmittel oder Verdünnungsmittelgemisch ausgewählt ist aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 20 Gew.-% daraus besteht.

11. Verfahren nach Anspruch 9 oder 10, **dadurch gekennzeichnet, dass** das Verfahren bei Temperaturen von mindestens 0 °C durchgeführt wird.

12. Verfahren nach einem der Ansprüche 9 bis 11, **dadurch gekennzeichnet, dass** das Gewichtsverhältnis der Gesamtmenge an Foramsulfuron, Mononatrium-Salz von Foramsulfuron, und Mono-Methanol-Solvat des Mononatrium-Salzes von Foramsulfuron nach Anspruch 8 zu der Gesamtmenge des in Schritt (a) eingesetzten Verdünnungsmittels oder oder Verdünnungsmittelgemisches kleiner als 2 : 1 ist.

13. Mischung, umfassend
(i) Mononatrium-Salz von Foramsulfuron nach einem der Ansprüche 1 bis 4 und/oder ein Solvat davon nach Anspruch 8, und
(ii) ein Verdünnungsmittel oder Verdünnungsmittelgemisch, ausgewählt aus der Gruppe bestehend aus Alkoholen mit 3 bis 6 C-Atomen, dabei vorzugsweise primären Alkoholen mit 3 bis 6 C-Atomen, und Ketonen mit 3 bis 6 C-Atomen, und deren Mischungen, oder zu mindestens 20 Gew.-% daraus besteht.

14. Agrochemische Zubereitung, enthaltend
(a) eine herbizid wirksame Menge des Mononatrium-Salzes von Foramsulfuron in kristalliner Form gemäß einem der Ansprüche 1 bis 4,
und ein oder mehrere weitere Bestandteile ausgewählt aus der Gruppe bestehend aus den nachfolgenden Bestandteilen (b-i), (b-ii) und (b-iii):
(b-i) im Pestizidbereich übliche Formulierhilfsstoffe,
(b-ii) weitere agrochemische Wirkstoffe,
und
(b-iii) dem Mono-Methanol-Solvat des Mononatrium-Salzes von Foramsulfuron nach Anspruch 8.

15. Verfahren zur Bekämpfung von Schadpflanzen oder zur Wachstumsregulierung von Pflanzen, **dadurch gekennzeichnet, dass** eine wirksame Menge
- des Mononatrium-Salzes von Foramsulfuron gemäß einem der Ansprüche 1 bis 4,
- einer Zusammensetzung nach Anspruch 5 oder 6, oder
- einer agrochemischen Zubereitung nach Anspruch 14
auf die Pflanzen, Pflanzensamen, den Boden, in dem oder auf dem die Pflanzen wachsen, oder die Anbaufläche appliziert wird.

## Claims

1. Monosodium salt of foramsulfuron in crystalline form, **characterized in that** the X-ray powder diffractogram of this salt using Cu Kα radiation at 25°C has at least 3 of the following 2Θ (2 theta) values:
| 28 (2 theta) values in ° |
|---|
| 6.2 |
| 6.4 |
| 14.4 |
| 14.6 |
| 14.7 |
| 18.4 |
| 19.2 |
| 20.1 |
| 23.2 |
| 24.7 |

2. Monosodium salt of foramsulfuron according to Claim 1, **characterized in that** the X-ray powder diffractogram of this salt using Cu Kα radiation at 25°C has at least 4, preferably at least 6, preferably at least 8, more preferably all of the 2Θ (2 theta) values specified in Claim 1.

3. Monosodium salt of foramsulfuron according to Claim 1, **characterized in that** the X-ray powder diffractogram of this salt using Cu Kα radiation at 25°C in addition has at least two, four, six, eight, ten, twelve or more of the following 2Θ (2 theta) values:
| |
|---|
| 10.3 |
| 10.5 |
| 11.1 |
| 11.3 |
| 11.4 |
| 12.0 |
| 12.1 |
| 12.4 |
| 12.6 |
| 12.9 |
| 13.6 |
| 14.0 |
| 15.0 |
| 15.1 |
| 15.4 |
| 16.0 |
| 16.2 |
| 17.0 |
| 17.2 |
| 18.2 |
| 18.8 |
| 20.3 |
| 20.6 |
| 20.8 |
| 21.2 |
| 21.6 |
| 22.7 |
| 23.7 |
| 24.6 |
| 25.4 |
| 25.9 |
| 26.2 |
| 26.9 |
| 27.1 |
| 27.7 |
| 28.3 |
| 29.0 |
| 30.2 |
| 30.5 |
| 30.7 |
| 31.0 |
| 31.2 |
| 31.9 |
| 32.5 |
| 32.6 |
| 33.7 |
| 34.8 |
| 35.6 |
| 35.9 |
| 36.2 |
| 36.4 |
| 36.9 |
| 37.4 |

4. Monosodium salt of foramsulfuron according to any of Claims 1 to 3, **characterized in that** the Raman spectrum thereof has at least the following bands:
| Band maximum [cm⁻¹] |
|---|
| 2959 |
| 2915 |
| 1686 |
| 1587 |
| 1526 |
| 694 |
| 345 |
| 133 |

5. Composition comprising the monosodium salt of foramsulfuron according to any of Claims 1 to 4 in a total amount of at least 5% by weight, based on the total amount of monosodium salt of foramsulfuron present in the composition.

6. Composition according to Claim 5, wherein
the composition is present in solid form at 25°C and 1013 mbar,
and/or
the content of monosodium salt of foramsulfuron is 80% by weight or more, based on the total weight of the composition.

7. Use of the monosodium salt of foramsulfuron according to any of Claims 1 to 4 or of a composition according to Claim 5 or 6 for preparing agrochemical formulations.

8. Monomethanol solvate of the monosodium salt of foramsulfuron in crystalline form, **characterized in that** the X-ray powder diffractogram of this salt using Cu Kα radiation at 25°C has at least 3 of the following 2Θ (2 theta) values:
| 2Θ (2 theta) values in ° |
|---|
| 9.1 |
| 9.1 |
| 9.7 |
| 12.6 |
| 16.2 |
| 18.5 |
| 24.1 |
| 24.3 |
| 24.6 |

9. Method for preparing the monosodium salt of foramsulfuron in crystalline form according to one or more of Claims 1 to 4, comprising the step of
(a) suspending a monosodium salt of foramsulfuron, the monomethanol solvate of the monosodium salt of foramsulfuron according to Claim 8, or a mixture of these 2 crystal forms, in a diluent or diluent mixture,
wherein the diluent or diluent mixture used in step (a) is selected from the group consisting of alcohols having 3 to 6 carbon atoms, in this case preferably primary alcohols having 3 to 6 carbon atoms, and ketones having 3 to 6 carbon atoms, and mixtures thereof, or comprises at least 20% by weight thereof.

10. Method for preparing the monosodium salt of foramsulfuron in crystalline form according to any of Claims 1 to 4, comprising the step of
(a) suspending foramsulfuron in a diluent or diluent mixture,
(b) reacting foramsulfuron with a base containing sodium, preferably an organic base, in this case preferably sodium methoxide and/or sodium ethoxide,
wherein the diluent or diluent mixture used in step (a) is selected from the group consisting of alcohols having 3 to 6 carbon atoms, in this case preferably primary alcohols having 3 to 6 carbon atoms, and ketones having 3 to 6 carbon atoms, and mixtures thereof, or comprises at least 20% by weight thereof.

11. Method according to Claim 9 or 10, **characterized in that** the method is carried out at a temperature of at least 0°C.

12. Method according to any of Claims 9 to 11, **characterized in that** the ratio by weight of the total amount of foramsulfuron, monosodium salt of foramsulfuron, and monomethanol solvate of the monosodium salt of foramsulfuron according to Claim 8, to the total amount of diluent or diluent mixture used in step (a) is less than 2:1.

13. Mixture comprising
(i) monosodium salt of foramsulfuron according to any of Claims 1 to 4 and/or a solvate thereof according to Claim 8, and
(ii) a diluent or diluent mixture selected from the group consisting of alcohols having 3 to 6 carbon atoms, in this case preferably primary alcohols having 3 to 6 carbon atoms, and ketones having 3 to 6 carbon atoms, and mixtures thereof, or comprises at least 20% by weight thereof.

14. Agrochemical formulation comprising
(a) a herbicidally effective amount of the monosodium salt of foramsulfuron in crystalline form according to any of Claims 1 to 4,
and one or more further constituents selected from the group consisting of the following constituents (b-i), (b-ii) and (b-iii):
(b-i) formulation auxiliaries customary in the pesticide field,
(b-ii) further agrochemical active ingredients, and
(b-iii) the monomethanol solvate of the monosodium salt of foramsulfuron according to Claim 8.

15. Method for controlling harmful plants or for regulating the growth of plants, **characterized in that** an effective amount
- of the monosodium salt of foramsulfuron according to any of Claims 1 to 4,
- a composition according to Claim 5 or 6, or
- an agrochemical formulation according to Claim 14 is applied to the plants, seeds of plants, the soil in which or on which the plants grow or the area under cultivation.

## Revendications

1. Sel monosodique de foramsulfuron sous forme cristalline, **caractérisé en ce que** le diagramme de diffraction des rayons X sur poudres de ce sel, par l'utilisation d'un rayonnement Cu K-α à 25°C, présente au moins 3 des valeurs à 2Θ (2 thêta) suivantes :
| valeurs à 2Θ (2 thêta) en ° |
|---|
| 6,2 |
| 6,4 |
| 14,4 |
| 14,6 |
| 14,7 |
| 18,4 |
| 19,2 |
| 20,1 |
| 23,2 |
| 24,7 |

2. Sel monosodique de foramsulfuron selon la revendication 1, **caractérisé en ce que** le diagramme de diffraction des rayons X sur poudres de ce sel, par l'utilisation d'un rayonnement Cu K-α à 25°C, présente au moins 4 valeurs à 2Θ (2 thêta), de préférence au moins 6 valeurs à 2Θ (2 thêta), préférablement au moins 8 valeurs à 2Θ (2 thêta), plus préférablement toutes les valeurs à 2Θ (2 thêta) mentionnées dans la revendication 1.

3. Sel monosodique de foramsulfuron selon la revendication 1, **caractérisé en ce que** le diagramme de diffraction des rayons X sur poudres de ce sel, par l'utilisation d'un rayonnement Cu K-α à 25°C, présente de plus au moins deux valeurs à 2Θ (2 thêta), au moins quatre valeurs à 2Θ (2 thêta), au moins six valeurs à 2Θ (2 thêta), au moins huit valeurs à 2Θ (2 thêta), au moins dix valeurs à 2Θ (2 thêta), au moins douze valeurs à 2Θ (2 thêta) ou plus, des valeurs à 2Θ (2 thêta) suivantes :
| |
|---|
| 10.3 |
| 10.5 |
| 11.1 |
| 11.3 |
| 11.4 |
| 12.0 |
| 12.1 |
| 12.4 |
| 12.6 |
| 12.9 |
| 13.6 |
| 14.0 |
| 15.0 |
| 15.1 |
| 15.4 |
| 16.0 |
| 16.2 |
| 17.0 |
| 17.2 |
| 18.2 |
| 18.8 |
| 20.3 |
| 20.6 |
| 20.8 |
| 21.2 |
| 21.6 |
| 22.7 |
| 23.7 |
| 24.6 |
| 25.4 |
| 25.9 |
| 26.2 |
| 26.9 |
| 27.1 |
| 27.7 |
| 28.3 |
| 29.0 |
| 30.2 |
| 30.5 |
| 30.7 |
| 31.0 |
| 31.2 |
| 31.9 |
| 32.5 |
| 32.6 |
| 33.7 |
| 34.8 |
| 35.6 |
| 35.9 |
| 36.2 |
| 36.4 |
| 36.9 |
| 37.4 |

4. Sel monosodique de foramsulfuron selon l'une quelconque des revendications 1 à 3, **caractérisé en ce que** son spectre Raman présente au moins les bandes suivantes :
| Maxima des bandes [cm⁻¹] |
|---|
| 2959 |
| 2915 |
| 1686 |
| 1587 |
| 1526 |
| 694 |
| 345 |
| 133 |

5. Composition, contenant le sel monosodique de foramsulfuron selon l'une quelconque des revendications 1 à 4 en une quantité totale d'au moins 5% en poids, par rapport à la quantité totale en sel monosodique de foramsulfuron contenue dans la composition.

6. Composition selon la revendication 5, la composition se trouvant sous forme solide à 25°C et à 1013 mbars
et/ou
la teneur en sel monosodique de foramsulfuron étant supérieure ou égale à 80%, par rapport au poids total de la composition.

7. Utilisation du sel monosodique de foramsulfuron selon l'une quelconque des revendications 1 à 4 ou d'une composition selon la revendication 5 ou 6 pour la préparation de préparations agrochimiques.

8. Solvate unique de méthanol du sel monosodique de foramsulfuron sous forme cristalline, **caractérisé en ce que** le diagramme de diffraction des rayons X sur poudres de ce sel, par l'utilisation d'un rayonnement Cu K-α à 25°C, présente au moins 3 des valeurs à 2Θ (2 thêta) suivantes :
| valeurs à 2Θ (2 thêta) en ° |
|---|
| 9,1 |
| 9,1 |
| 9,7 |
| 12,6 |
| 16,2 |
| 18,5 |
| 24,1 |
| 24,3 |
| 24,6 |

9. Procédé pour la préparation du sel monosodique de foramsulfuron sous forme cristalline selon l'une quelconque des revendications 1 à 4, comprenant l'étape de
(a) suspension d'un sel monosodique de foramsulfuron, du solvate unique de méthanol du sel monosodique de foramsulfuron selon la revendication 8 ou d'un mélange de ces deux formes cristallines dans un diluant ou dans un mélange de diluants,
le diluant ou le mélange de diluants utilisé dans l'étape (a) étant choisi dans le groupe constitué par les alcools comprenant 3 à 6 atomes de C, de préférence les alcools primaires comprenant 3 à 6 atomes de C et les cétones comprenant 3 à 6 atomes de C et les mélanges de ceux-ci ou bien est constitué d'au moins 20% en poids de ceux-ci.

10. Procédé pour la préparation du sel monosodique de foramsulfuron sous forme cristalline selon l'une quelconque des revendications 1 à 4, comprenant l'étape de
(a) suspension du foramsulfuron dans un diluant ou dans un mélange de diluants,
(b) transformation du foramsulfuron avec une base contenant du sodium, de préférence une base organique, préférablement avec du méthanolate de sodium et/ou avec de l'éthanolate de sodium,
le diluant ou le mélange de diluants utilisé dans l'étape (a) étant choisi dans le groupe constitué par les alcools comprenant 3 à 6 atomes de C, de préférence les alcools primaires comprenant 3 à 6 atomes de C et les cétones comprenant 3 à 6 atomes de C et les mélanges de ceux-ci ou bien est constitué d'au moins 20% en poids de ceux-ci.

11. Procédé selon la revendication 9 ou 10, **caractérisé en ce que** le procédé est mis en oeuvre à des températures d'au moins 0°C.

12. Procédé selon l'une quelconque des revendications 9 à 11, **caractérisé en ce que** le rapport pondéral de la quantité totale de foramsulfuron, du sel monosodique de foramsulfuron et du solvate unique de méthanol du sel monosodique de foramsulfuron selon la revendication 8 à la quantité totale du diluant ou du mélange de diluants utilisé dans l'étape (a) est inférieur à 2:1.

13. Mélange, comprenant
(i) le sel monosodique de foramsulfuron selon l'une quelconque des revendications 1 à 4 et/ou un solvate correspondant selon la revendication 8 et
(ii) un diluant ou un mélange de diluants choisi dans le groupe constitué par les alcools comprenant 3 à 6 atomes de C, de préférence les alcools primaires comprenant 3 à 6 atomes de C et les cétones comprenant 3 à 6 atomes de C et les mélanges de ceux-ci ou bien est constitué d'au moins 20% de ceux-ci.

14. Préparation agrochimique contenant
(a) une quantité active en tant qu'herbicide du sel monosodique de foramsulfuron sous forme cristalline selon l'une quelconque des revendications 1 à 4,
et un ou plusieurs autre(s) ingrédient(s) choisi(s) dans le groupe constitué par les ingrédients suivants (b-i), (b-ii) et (b-iii):
(b-i) des adjuvants de formulation habituels dans le domaine des pesticides,
(b-ii) d'autres principes actifs agrochimiques,
et
(b-iii) le solvate unique de méthanol du sel monosodique de foramsulfuron selon la revendication 8.

15. Procédé pour la lutte contre les plantes nuisibles ou pour la régulation de la croissance de plantes, **caractérisé en ce qu'**une quantité active
- du sel monosodique de foramsulfuron selon l'une quelconque des revendications 1 à 4,
- d'une composition selon la revendication 5 ou 6, ou
- d'une préparation agrochimique selon la revendication 14
est appliquée sur les plantes, sur les semences de plantes, sur le sol dans lequel ou sur lequel les plantes poussent ou sur la zone cultivée.
